# EUROPÄISCHE PATENTSCHRIFT

(11) **EP 3 038 751 B1**
(45) Veröffentlichungstag und Bekanntmachung des Hinweises auf die Patenterteilung: **04.10.2017**
(21) Anmeldenummer: 14750215.7
(22) Anmeldetag: 08.08.2014
(51) Int. Cl.: B01J 31/18, B01J 21/02, B01J 21/06, B01J 21/08, B01J 21/18, C07C 45/50, B01J 8/02

(54) **GETRÄGERTE ZUSAMMENSETZUNG UND DEREN VERWENDUNG IN VERFAHREN ZUR HYDROFORMYLIERUNG VON UNGESÄTTIGTEN VERBINDUNGEN**
SUPPORTED COMPOSITION AND THE USE THEREOF IN METHODS FOR THE HYDROFORMYLATION OF UNSATURATED COMPOUNDS
COMPOSITION SUR SUPPORT ET SON UTILISATION DANS DES PROCÉDÉS D'HYDROFORMYLATION DE COMPOSÉS INSATURÉS

(30) Priorität: 28.08.2013 DE 102013217174
(43) Veröffentlichungstag der Anmeldung: 06.07.2016
(73) Patentinhaber: Evonik Degussa GmbH, 45128 Essen (DE)
(72) Erfinder: DYBALLA, Katrin Marie, 45657 Recklinghausen (DE); HAHN, Hanna, 47199 Duisburg-Baerl (DE); FRANKE, Robert, 45772 Marl (DE); FRIDAG, Dirk, 45721 Haltern am See (DE); BECKER, Marc, 44359 Dortmund (DE)
(86) Internationale Anmeldenummer: PCT/EP2014/067061
(87) Internationale Veröffentlichungsnummer: WO 2015/028281

(56) Entgegenhaltungen:
- WO-A1-2014/056736
- DE-A1-102008 002 188
- US-A- 5 767 321
- US-A- 5 962 744
- US-A1- 2012 190 894

## Beschreibung

Die vorliegende Erfindung ist gerichtet auf eine Zusammensetzung, umfassend: a) mindestens ein Trägermaterial; b) mindestens ein Metall, ausgewählt aus der VIII. Nebengruppe des Periodensystems der Elemente und c) mindestens eine Verbindung der Formel (I) R'-A-R" (I), wobei A, R' und R" jeweils ein organischer Rest, wobei R' und R" das Strukturelement -O-P(-O-)₂ mit dreiwertigem P aufweisen und über dieses mit dem Rest A kovalent verknüpft sind, mit der Maßgabe, dass R' ≠ R" ein Verfahren zur Herstellung einer solchen Zusammensetzung, die Verwendung der Zusammensetzung sowie ein Verfahren und eine Vorrichtung zur Hydroformylierung, bei der die Zusammensetzung verwendet wird.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt (Schema 1). Als Katalysatoren in diesen Reaktionen werden häufig Verbindungen der Übergangsmetalle der VIII. Gruppe des Periodensystems der Elemente verwendet, insbesondere Rhodium- oder Cobaltkatalysatoren. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

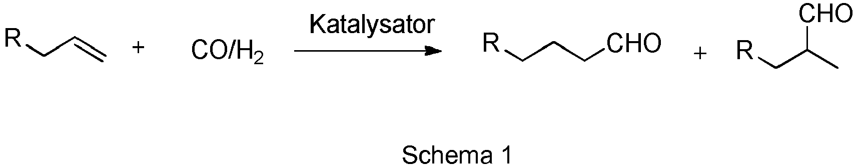

In der klassischen homogenen Katalyse, speziell der Hydroformylierung, werden wie in US 4769498 und US 5723641 beschrieben, bevorzugt symmetrisch aufgebaute Bisphosphite als Liganden verwendet. Unsymmetrisch aufgebauten Bisphosphite weisen bei der Verwendung als Liganden in der übergangsmetallkatalysierten Hydroformylierung deutlich geringere Reaktivitäten und geringere n-Regioselektivität auf; siehe in Rhodium-catalyzed Hydroformylation, ed. by P.W.N.M. van Leeuwen et C. Claver, Kluwer Academic Publishers 2006, AA Dordrecht, NL, Seite 45-46.

Wie von van Leeuwen ausführt, weisen die symmetrischen Bisphosphite neben höheren n/iso-Selektivitäten auch eine größere Reaktivität auf. Neben dem Bestreben einer hohen Reaktivität und n/iso-Selektivität in Bezug auf die zu carbonylierenden, ungesättigten Verbindungen ist die Stabilität - konkret die Standzeit - der katalytisch aktiven Zusammensetzung aus jeweils verwendetem Metall, Liganden sowie weiteren Komponenten mit aktivierender Wirkung mit Blick auf die als Liganden eingesetzten Bisphophite eine ständige Aufgabe der Forschung. Dies gilt insbesondere hinsichtlich olefinhaltiger Gemische, speziell in der Hydroformylierung von Gemischen linearer Olefine.

Aldehyde, insbesondere lineare Aldehyde wie Butyraldehyd, Valeraldehyd, Hexanal bzw. Octanal haben technische Bedeutung als Ausgangsprodukte für Weichmacheralkohole, Tenside und Feinchemikalien.

Insgesamt wurden im Jahr 2008 mehr als 8 Mio. Tonnen Oxo-Produkte mittels Hydroformylierung produziert.

Katalysatoren, die im Rahmen der Hydroformylierungsreaktion allgemein verwendet werden, sind insbesondere Rhodium- und Cobaltverbindungen in Gegenwart von Liganden. Heutzutage werden in den Hydroformylierungsprozessen vor allem homogen gelöste Rhodium-basierte Organometallkatalysatoren eingesetzt, da hierbei im Gegensatz zu den Cobalt-basierten Verfahren deutlich mildere Reaktionsbedingungen gewählt werden können (siehe: H.-W. Bohnen, B. Cornils, Adv. Catal. 2002, 47, 1).

Die Hydroformylierung von Olefinen unter Verwendung von Rhodium aufweisenden Katalysatorsystemen wird im Wesentlichen nach zwei Grundvarianten durchgeführt.

In einer, dem Ruhrchemie/Rhone-Poulenc-Verfahren, ist das Katalysatorsystem, bestehend aus Rhodium und einem wasserlöslichen Ligand, meistens Alkalimetallsalze von sulfonierten Phosphinen, in einer wässrigen Phase gelöst. Das Edukt-Produkt-Gemisch bildet eine zweite flüssige Phase. Die beiden Phasen werden durch Rühren vermischt und von Synthesegas und Olefin, falls gasförmig, durchströmt. Die Abtrennung des Edukt-Produkt-Gemischs vom Katalysatorsystem erfolgt durch Phasentrennung. Die abgetrennte organische Phase wird destillativ aufgearbeitet (siehe: C. W. Kohlpaintner, R. W. Fischer, B. Cornils, Appl. Catal. A Chem. 2001, 221, 219).

Nachteilig an diesem Verfahren ist neben dem hohen Kapitaleinsatz und den hohen Betriebskosten, dass nur gegen Wasser stabile Liganden eingesetzt werden können und dass Rhodiumverluste durch Auslaugung nicht vermeidbar sind. Dies ist besonders problematisch, da gerade Rhodium-Verbindungen vergleichsweise teure Edelmetall-Komplexe darstellen, da Rhodium zu den teuersten Metalen überhaupt zählt.

In der anderen Variante ist das Rhodium aufweisende Katalysatorsystem in einer organischen Phase homogen gelöst. In diese Phase wird Synthesegas und Einsatzolefin eingeleitet. Das aus dem Reaktor abgezogene Reaktionsgemisch wird durch zum Beispiel Destillation oder Membrantrennung in eine Produkt-Eduktphase und eine Hochsiederphase, die das Rhodium aufweisende, Katalysatorsystem gelöst enthält, getrennt. Die das Rhodium aufweisende Katalysatorsystem enthaltende Phase wird in den Reaktor zurückgeführt, die andere Phase wird destillativ aufgearbeitet (siehe: K.-D. Wiese, D. Obst, Hydroformylation in: Catalytic Carbonylation Reactions; M. Beller (Ed.), Topics in Organometallic Chemistry 18, Springer, Heidelberg, Germany, 2006, 1).

Bei der Hydroformylierung entstehen Hochsieder. Zum größten Teil handelt es sich um Aldoladditions- oder Aldolkondensationsprodukte aus den gebildeten Aldehyden. Damit die Hochsiederkonzentration im Reaktor begrenzt bleibt, muss ein Teilstrom, möglichst einer, in dem die Hochsieder aufkonzentriert sind, ausgeschleust werden. In diesem Teilstrom sind Rhodiumverbindungen enthalten. Um die Rhodiumverluste klein zu halten, muss Rhodium aus diesem Ausschleusestrom zurückgewonnen werden. Die Rhodiumabtrennung aus solchen Strömen ist nicht vollständig und aufwendig. Weitere Rhodiumverluste treten durch Cluster-Bildung des Rhodiums auf. Diese Rhodium-Cluster lagern sich an Gerätewände ab und bilden ggf. mit den Gerätematerialien Legierungen. Diese Rhodiummengen sind nicht mehr katalytisch wirksam und können auch nach Abstellung der Betriebsanlage nur sehr aufwendig und auch nur teilweise zurückgewonnen werden.

Da wegen des außergewöhnlich hohen Rhodiumpreises in den letzten Jahren die Wirtschaftlichkeit eines technischen Hydroformylierungsverfahren weitgehend vom spezifischen Rhodiumverbrauch abhängig ist, wurde versucht, Alternativverfahren zu entwickeln, die sich durch geringere spezifische Rhodiumverluste auszeichnen.

Bei der Entwicklung neuer Hydroformylierungsverfahren wurde von der Idee ausgegangen, die bislang homogen in der Reaktionsmischung vorliegenden, Rhodium aufweisenden Katalysatorsysteme zu immobilisieren. Es kann in diesem Zusammenhang von der Heterogenisierung einer an sich homogen durchgeführten Reaktion - in diesem Fall der Hydroformylierung - gesprochen werden.

In den letzten Jahrzehnten sind zahlreiche Techniken zur Immobilisierung homogener Katalysatoren entwickelt und viele dieser Konzepte für Hydroformylierungsreaktion angewendet worden (siehe: M. Beller, B. Cornils, C. D. Frohning, C. W. Kohlpaintner, J. Mol. Catal. 1995, 104, 17).

Die Heterogenisierung der Katalysatorkomplexe durch Immobilisierung auf porösen Trägermaterialien ist eingehend untersucht worden. Solche Heterogenisierung kann z. B. durch kovalente Verankerung des Rhodium-Komplexes über Spacer-Liganden auf dem Träger erreicht werden (siehe: V. A. Likholobov, B. L. Moroz, Hydroformylation on Solid Catalysts in: Handbook of Heterogeneous Catalysis, 2nd ed.; G. Ertl, H. Knoezinger, F. Schüth, J. Weitkamp (Eds.), Wiley-VCH, Weinheim, Germany, 2008, 3663).

Neben dem Supported-Aqueous-Phase (SAP) Konzept (siehe H. Delmas, U. Jaeuregui-Haza, A.-M. Wilhelm, Supported Aqueous-Phase Catalysis as the Alternative Method in: Multiphase Homogeneous Catalysis, B. Cornils, W. A. Herrmann, I. T. Horväth, W. Leitner, S. Mecking, H. Olivier-Bourbigou, D. Vogt (Eds.), Wiley-VCH, Weinheim, Germany, 2005**),** welches aber für hydrolyseempfindliche Liganden ungeeignet ist, stellt das sogenannte Supported-Liquid-Phase (SLP) Konzept ein weiteres Konzept zur Heterogenisierung von homogenen Katalysatorkomplexen dar: Hierbei wird eine flüssige Katalysatorlösung auf ein poröses Trägermaterial aufgebracht. Dieses Konzept ist bereits über 40 Jahre bekannt (siehe: P. Rony, J. Catal. 1969, 14, 142; G.J.K. Acres, G.C. Bond, B.J. Cooper, J.A. Dawson, J. Catal. 1969, 6, 139). Für die Hydroformylierung werden unter anderem geschmolzene Salze wie z.B. Triphenylphosphan (TPP) als flüssige Phase eingesetzt. TPP dient hierbei als Lösungsmittel für den Katalysatorkomplex, aber auch als Ligand und wird daher in einem großen Überschuss eingesetzt. Problematisch an einem sehr großen Ligandenüberschuss bei betrachteten Katalysatorsystemen ist die Bildungen verschiedener Übergangsmetallkomplexe, die eine Unterdrückung der katalytischen Aktivität zur Folge haben können.

Nach Literaturangaben leiden jedoch rein heterogene Katalysatoren unter einer niedrigen Hydroformylierungsaktivität, zeichnen sich jedoch durch eine in diesem Fall unerwünschte recht hohe Hydrieraktivität aus (siehe: a) M. E. Davis, E. Rode, D. Taylor, B. E. Hanson, J. Catal.1984, 86, 67; b) S. Naito, M. Tanimoto, J. Chem. Soc. Chem. Commun. 1989, 1403; c) G. Srinivas, S. S. C. Chung, J. Catal. 1993, 144, 131). Ohne das Vorhandensein einer flüssigen Reaktionsphase, in der der metallorganische Katalysatorkomplex gelöst vorliegt, wird oft eine schlechte Regioselektivität festgestellt.

Die bislang aussichtsreichste Entwicklung ist die Hydroformylierung von Olefinen zu Aldehyden mittels sogenannter Supported-Ionic-Liquid-Phase-, kurz genannt SILP-Katalysatorsystemen. Dies sind katalytisch wirksame Zusammensetzungen in einem Mehrphasensystem, die aus einem festen, inerten, porösen Trägermaterial bestehen, das mit einer ionischen Flüssigkeit umhüllt ist - der sogenannten SILP-Phase - in welcher der Übergangsmetall-, insbesondere Rhodium, aufweisende Katalysator enthalten ist (siehe: a) A. Riisager, P. Wasserscheid, R. van Hal, R. Fehrmann, J. Catal. 2003, 219, 252; b) M. Haumann, K. Dentler, J. Joni, A. Riisager, P. Wasserscheid, Adv. Synth. Catal. 2007, 349, 425; c) S. Shylesh, D. Hanna, S. Werner, A. T. Bell, ACS Catal. 2012, 2, 487; d) M. Jakuttis, A. Schoenweiz, S. Werner, R. Franke, K.-D. Wiese, M. Haumann, P. Wasserscheid, Angew. Chem. lnt. Ed. 2011, 50, 4492).

Mit SILP-Katalysatorsystemen lassen sich die Vorteile von homogen und heterogen katalysierten Synthesereaktionen vereinen. Dies betrifft vor allem die Produktabtrennung und Rückgewinnung des Katalysators, insbesondere der darin enthaltenen Übergangsmetalle, welche sich bei homogen geführten Synthesereaktionen als schwierig und aufwendig darstellt. Bei heterogen katalysierten Synthesereaktionen kann es hingegen zu Massen- und Wärmetransportlimitierung kommen, wodurch sich die Aktivität des festen Katalysatorsystems verringert; auch werden bei heterogen katalysierten Synthesereaktionen geringere Chemo- und Stereoselektivitäten beobachtet.

Für den wirtschaftlichen Betrieb eines kontinuierlichen Verfahrens zur Hydroformylierung ist nicht allein die Nutzung eines sehr aktiven und selektiven Katalysatorsystems von Bedeutung. Besonders die Punkte Katalysatorrecycling - verbunden mit der Produktabtrennung - und Ligandenstabilität spielen eine entscheidende Rolle - nicht nur angesichts der hohen Rhodium- und Ligandenpreise, sondern auch des nur ansatzweise bekannten Einflusses von Verunreinigungen aus Liganden-Abbauprozessen auf die Aktivität und das Produktspektrum.

Nachteilig bei dem beschriebenen SILP-Verfahren ist hierbei die Verwendung der ionischen Flüssigkeit, kurz IL genannt; die langfristige Toxizität dieser ionischen Flüssigkeiten ist z.T. noch ungeklärt bzw. es hat sich erwiesen, dass einige mögliche Kationen und Anionen ökotoxisch sind. So weisen unter anderem längere Alkylketten eine Aquatoxizität auf. Zwei weitere Probleme sind die noch zu hohen Produktionskosten und die bei vielen ionischen Flüssigkeiten mangelnde Beständigkeit gegenüber höheren Temperaturen.

Hinzukommt, dass die kommerziell verfügbaren ILs aufgrund ihrer Synthese in den meisten Fällen Spuren oder sogar größere Mengen an Wasser enthalten können. Das Trocknen dieser Ionischen Flüssigkeiten ist in der Regel sehr aufwendig und problematisch, da es nicht in allen Fällen gelingt.

Der zusätzliche Eintrag von Wasser über die Ionische Flüssigkeit ist besonders kritisch, da allgemein bekannt ist, dass Organophosphor-Liganden in der Hydroformylierung einem inhärenten Abbau- und Desaktivierungsprozess unterliegen. [P. W. N. M. van Leeuwen, in Rhodium Catalyzed Hydroformylation, P. W. N. M. van Leeuwen, C. Claver (Hrsg.), Kluwer, Dordrecht, 2000.]

Neben- und Abbaureaktionen können beispielsweise Hydrolyse, Alkoholyse, Umesterung, Arbusov-Umlagerung, P-O-Bindungsspaltung und P-C-Bindungsspaltung sein [P. W. N. M. van Leeuwen, in Rhodium Catalyzed Hydroformylation, P. W. N. M. van Leeuwen, C. Claver (Hrsg.), Kluwer, Dordrecht, 2000.; F. Ramirez, S. B. Bhatia, C. P. Smith, Tetrahedron 1967, 23, 2067-2080.; E. Billig, A. G. Abatjoglou, D. R. Bryant, R. E. Murray, J. M. Maher, (Union Carbide Corporation), US Pat. 4,789,753 1988; M. Takai, I. Nakajima, T. Tsukahara, Y. Tanaka, H. Urata, A. Nakanishi, EP 1 008 581 B1 2004.].

Liganden-Desaktivierung und -Abbau führen dazu, dass weniger aktiver Ligand im System vorhanden ist, was sich nachteilig auf die Performance des Katalysators auswirken kann (Umsatz, Ausbeute, Selektivität).

Somit sollte ein zusätzlicher Eintrag von Stoffen, die diesen Katalysatorabbau beschleunigen, wie beispielsweise ein Wassereintrag über die IL, vermieden werden.

Literaturbekannte Ligandensysteme für die Gasphasenhydroformylierung mit SILP-Systemen sind u.a. Bisphosphite (DE 102010041821), Mono- und Bisphosphine (Riisager et al., Catal. Lett. 2003, 90, 149.; Riisager et al. J. Catal. 2003, 219, 452). Bisphosphite zeichnen sich durch eine hohe Langzeitstabilität von 700 h bei einer Reaktionstemperatur von 100°C aus. SILP-Katalysatoren ohne IL können aus oben genannten Gründen längere Standzeiten von bis zu 1000 h und n/iso-Selektivitäten größer 95% erzielt (Schönweiz et al., Chem. Cat. Chem., 2013, DOI: 10.1002/cctc.201300305). Sulfonierten Bisphosphinen des Xantphostyps als SILP-Katalysator ohne IL zeigen gegenüber Systemen mit IL ([BMIM][PF₆]) in einer fünfstündigen Hydroformylierungsreaktionen von Propen eine deutlich gesteigerte Aktivität (TOF(L/Rh-Verhältnis = 2,5) = 37,4 h⁻¹ ohne IL zu 5,1 h⁻¹ mit [BMIM][PF₆]; bzw. TOF(L/Rh-Verhältnis = 10,2 ohne IL bzw. 10 mit [BMIM][PF₆]) = 40,8 h⁻¹ ohne IL zu 37,0 h⁻¹ mit [BMIM][PF₆]) bei einer nur geringfügig kleineren n/iso-Selektivität (n/iso-Selektivität (L/Rh-Verhältnis = 2,5) = 1,7% ohne IL zu 2,0 % mit [BMIM][PF₆]; bzw. TOF(L/Rh-Verhältnis = 10,2 ohne IL bzw. 10 mit [BMIM][PF₆]) = 16,9% ohne IL zu 23,3% mit [BMIM][PF₆]) (Riisager et al., J. Catal. 2003, 219, 452). Auch Rh-Monophosphine (Riisager et al., Catal. Lett. 2003, 90, 149.) liefern in Propen-Hydroformylierungsreaktionen als SILP-Katalysator ohne IL's eine deutlich höhere Aktivität (Turn over frequency) sowie vergleichbare n/iso-Selektivität.

In vielen technischen Strömen sind neben terminalen Alkenen häufig auch interne Olefine zu finden. Daher ist es wichtig eine katalytisch aktive Zusammensetzung zu entwickeln, die neben der Umsetzung von terminalen Alkenen auch zur Umsetzung in einer isomerisierenden Hydroformylierung geeignet ist. Die literaturbekannten Liganden des Benzpinacol-Typs sind jedoch nicht in einer isomerisierenden Hydroformylierung aktiv, wie bereits DE 102006058682 offenbart.

Dieser Ligandentyp stellt das bisher stabilste System einer katalytisch aktiven Zusammensensetzung in der Heterogenisierung der homogenen Hydroformylierungsreaktion dar, wie bereits DE 102010041821 offenbart.

Es ist in diesem Zusammenhang wünschenswert, katalytisch aktive Zusammensetzungen zu entwickeln, die isomerisierend hydroformylieren können und gleichzeitig deutlich stabiler als der bisherige Stand der Technik sind, d.h. sich durch eine deutlich längere Laufzeit auszeichnen.

Für einen großtechnischen Hydroformylierungsprozess ist neben der n/iso-Selektivität > 90 % aber auch die Standzeit eines Katalysators von entscheidender Bedeutung. Pro Katalysatorwechsel sind Rüstzeiten der Produktionsanlage notwendig, in denen die Produktionsanlage nicht in Betrieb ist. In diesen Zeiten kann mit dieser Produktionsanlage kein Ertrag erwirtschaftet werden.

Aufgabe der vorliegenden Erfindung war die Bereitstellung eines Katalysatorsystems für die Hydroformylierung welches einen oder mehrere der Nachteile der Katalysatorsysteme des Standes der Technik nicht aufweist.

Insbesondere war Aufgabe der vorliegenden Erfindung ein Verfahren zur Hydroformylierung bereitzustellen, welches sowohl eine günstige Katalysatorabtrennung ermöglicht, als auch auf den Zusatz weiterer Komponenten verzichtet und vorzugsweise gleichzeitig eine verbesserte Katalysatorstandzeit im Vergleich zu den im Stand der Technik beschriebenen Systemen aufweist.

Überraschenderweise wurde gefunden, dass diese Aufgabe gelöst werden kann durch eine Zusammensetzung gemäß Anspruch 1, die einen Katalysatorkomplex auf einem heterogenen Träger aufweist, wobei der Komplex einen unsymmetrisch substituierten Bisphosphit-Liganden aufweist.

Die erfindungsgemäße Zusammensetzung hat den Vorteil, dass beispielsweise auf den Zusatz einer IL, wie es ein SILP-Katalysatorsystem vorsieht, verzichtet werden kann. Dadurch können zum einen Kosten zur Synthese der IL oder deren Beschaffung eingespart werden; zum anderen kann die Einschleusung von Katalysatorgiften wie Wasser über die IL vermieden werden.

Ein weiterer Vorteil der erfindungsgemäßen Zusammensetzung sind die langen Katalysatorstandzeiten, insbesondere bei Anwendungen der Zusammensetzung als Katalysator in Gasphasenreaktionen, da auf einen mehrfachen Katalysatorwechsel im Laufe eines Betriebsjahres verzichtet werden kann. So führt die Heterogenisierung eines homogenen Ligandenkomplexes durch ein langzeitstabiles SILP-System ohne einen Zusatz oder ohne die Anwesenheit von Ionischen Flüssigkeiten auf porösen Trägermaterialien zu Standzeiten von mehr als 3500 h und zu n/iso-Selektivitäten von durchschnittlich mehr als 90%. Dies bedeutet zugleich eine vereinfachte Produktabtrennung und Rückgewinnung des Katalysators, insbesondere der darin enthaltenen Übergangsmetalle gegenüber homogenen Systemen, da ein Festbettreaktor verwendet werden kann.

Ein weiterer Vorteil der erfindungsgemäßen Zusammensetzung besteht darin, dass diese neben der Umsetzung von terminalen Alkenen auch die Umsetzung in einer isomerisierenden Hydroformylierung katalysiert.

Die Eignung des erfindungsgemäßen Katalysatorsystem für die Hydroformylierung ist insbesondere deshalb überraschend, da unsymmetrisch aufgebaute Bisphosphite im allgemeinen bei der Verwendung als Ligand in der übergangsmetallkatalysierten Hydroformylierung in der Flüssigphase deutlich geringere Reaktivitäten und geringere n-Regioselektivität als symmetrisch substituierte Bisphosphit-Liganden aufweisen; siehe in Rhodium-catalyzed Hydroformylation, ed. by P.W.N.M. van Leeuwen et C. Claver, Kluwer Academic Publishers 2006, AA Dordrecht, NL, Seite 45-46. Der in dem beanspruchten System verwendete unsymmetrische Bisphosphitligand weist die im Stand der Technik beschriebenen Nachteile nicht auf. Im Vergleich zu allen im Stand der Technik genannten heterogenisierten symmetrischen Bisphosphiten weist die erfindungsgemäße Zusammensetzung die mit Abstand beste Katalysatorstandzeit auf und zeichnet sich damit durch hohe Stabilität aus.

Die erfindungsgemäßen Zusammensetzungen sowie deren Verwendung werden nachfolgend beispielhaft beschrieben, ohne dass die Erfindung auf diese beispielhaften Ausführungsformen beschränkt sein soll. Sind nachfolgende Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Mit dem Begriff "inert" wird im Sinne der vorliegenden Erfindung die Eigenschaft von Stoffen, Komponenten oder Gemischen verstanden, welche sich dadurch auszeichnet, dass sich keine nachteiligen Auswirkungen oder dem beabsichtigten Reaktionsablauf gegenläufige Effekte einstellen.

Die erfindungsgemäße Zusammensetzung zeichnet sich dadurch aus, dass sie umfasst:
a) mindestens ein Trägermaterial, welches vorzugsweise porös ist;
b) mindestens ein Metall, ausgewählt aus der VIII. Nebengruppe des Periodensystems der Elemente;
c) mindestens eine Verbindung der Formel (II)

Als Trägermaterialien können alle bekannten Trägermaterialien, vorzugsweise porösen Trägermaterialien eingesetzt werden. Vorzugsweise werden als poröse Trägermaterialien solche eingesetzt, die inert bezüglich der weiteren Bestandteile der Zusammensetzung und den Reaktionspartnern und -produkten der Reaktionen, bei denen die Zusammensetzungen eingesetzt werden. Bevorzugte Trägermaterialien sind anorganisehe, vorzugsweise oxidische Trägermaterialien. Als Trägermaterialien eigenen sich insbesondere Oxide von Aluminium, Silizium, Titan, Zirkon oder Aktivkohle oder Mischungen davon, die gegebenenfalls noch weitere Elemente aufweisen können. Bevorzugte Trägermaterialien sind z. B. Alumosilikate, Zeolithe, Al₂O₃ oder Siliziumdioxid. Besonders bevorzugt weist das Trägermaterial Siliziumdioxid auf oder besteht daraus.

Das poröse Trägermaterial weist vorzugsweise die Oberflächenparameter:
i) mittlerer Porendurchmesser in einem Bereich von 1 bis 423 nm;
ii) Porenvolumen in einem Bereich von 0,1 bis 2 ml/g;
iii) BET-Oberfläche in einem Bereich von 10 bis 2050 m²/g auf, wobei die Bestimmung dieser Werte nach der Hg-Methode gemäß DIN 66133 sowie der N₂-Adsorption nach DIN 66131 und DIN 66135 erfolgt.

Die Verbindung der Formel (II) in der erfindungsgemäßen Zusammensetzung ist: 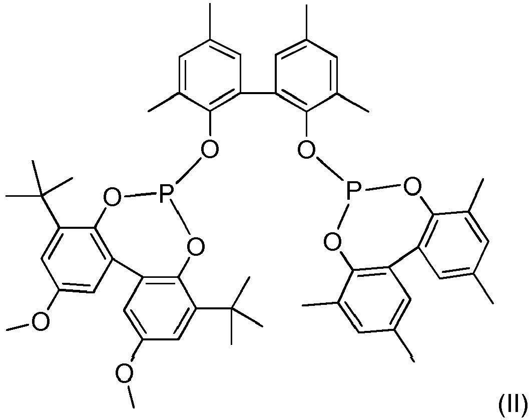 wobei die Verbindung der Formel (II) weitere Bestandteile aufweisen kann, welche auf Verunreinigungen in der Synthese von (II), wie z.B. unumgesetztes Edukt, Hydrolyse-, Oxidationsprodukte von (II) oder weitere Verunreinigungen, zurückzuführen sind.

In der erfindungsgemäßen Zusammensetzung ist das Metall vorzugsweise ausgewählt unter:
Cobalt, Rhodium, Iridium, Ruthenium, insbesondere Rhodium.

Die erfindungsgemäße Zusammensetzung kann auf jede bekannte Weise durch Mischen der Komponenten hergestellt werden. Vorzugsweise wird die erfindungsgemäße Zusammensetzung durch das nachfolgend beschriebene erfindungsgemäße Verfahren hergestellt bzw. ist dadurch erhältlich.

Das erfindungsgemäße Verfahren zur Herstellung der erfindungsgemäßen Zusammensetzung, zeichnet sich dadurch aus, dass es die Schritte aufweist:
a) vorlegen einer Vorstufe mindestens einer Verbindung eines Metalls aus der VIII. Nebengruppe des Periodensystems der Elemente;
b) in-Kontakt-bringen mindestens einer Verbindung eines Metalls aus der VIII. Nebengruppe des Periodensystems der Elemente mit einem molaren Überschuss an mindestens einer Phosphorhaltigen organischen Verbindung der Formel (II) unter Verwendung eines inerten Lösungsmittels;
c) Zugabe mindestens eines porösen, inerten Trägermaterials zu der unter b) generierten Mischung;
d) entfernen des inerten Lösungsmittels unter Erhalt der katalytisch aktiven Zusammensetzung;
wobei bevorzugt die Schritte a) bis c) in beliebiger Reihenfolge durchgeführt und wobei bevorzugt in Schritt a) mindestens eine Verbindung eines Metalls aus der VIII. Nebengruppe in einem inerten Lösungsmittel vorgelegt werden kann.

Die erfindungsgemäße Zusammensetzung kann als katalytisch aktive Zusammensetzung eingesetzt werden. Vorzugsweise wird die erfindungsgemäße Zusammensetzung als katalytisch aktive Zusammensetzung in einem Verfahren zur Hydroformylierung von ungesättigten Verbindungen oder Gemischen davon verwendet.

Das erfindungsgemäße Verfahren zur Hydroformylierung von ungesättigten Verbindungen zeichnet sich demgemäß dadurch aus, dass eine erfindungsgemäße Zusammensetzung als Katalysator eingesetzt wird. Bevorzugt wird das erfindungsgemäße Verfahren zur Hydroformylierung von ungesättigten Verbindungen oder Gemischen davon in einem Festbettreaktor durchgeführt, der die erfindungsgemäße Zusammensetzung enthält.

Die ungesättigten Verbindungen sind vorzugsweise ausgewählt aus C₂ - C₄ - Olefinen und deren technischen Gemischen, wie sie z.B. als Raffinatströme - Raffinat I, II oder III - bei der Auf- und Weiterverarbeitung in der petrochemischen Industrie vorliegen, insbesondere Ethen, Propen, Butene oder Gemische, die diese Verbindungen aufweisen.

Ein weiterer Gegenstand der vorliegenden Erfindung ist eine Vorrichtung zur Hydroformylierung von ungesättigten Verbindungen, welche als Festbettreaktor betrieben wird, und dadurch gekennzeichnet ist, dass sie eine erfindungsgemäße Zusammensetzung, vorzugsweise im oder als Festbett, aufweist.

Abschließender Gegenstand der vorliegenden Erfindung ist ein mehrphasiges Reaktionsgemisch, beinhaltend:
1) mindestens eine ungesättigte Verbindung;
2) ein Gasgemisch, umfassend Kohlenmonoxid, Wasserstoff sowie
3) Aldehyde und ihre Folgeprodukte, in Gegenwart der erfindungsgemäßen Zusammensetzung.

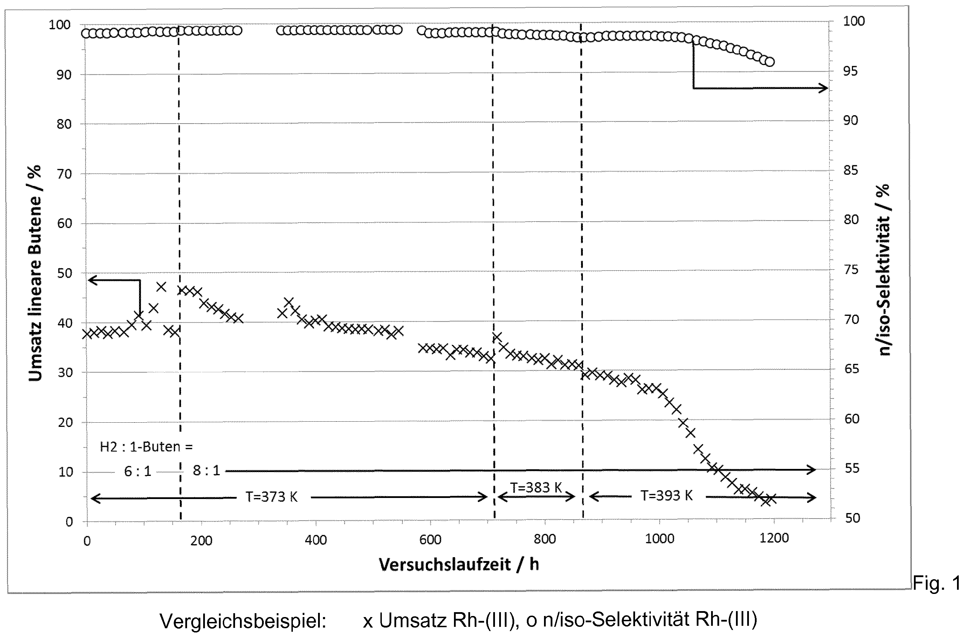

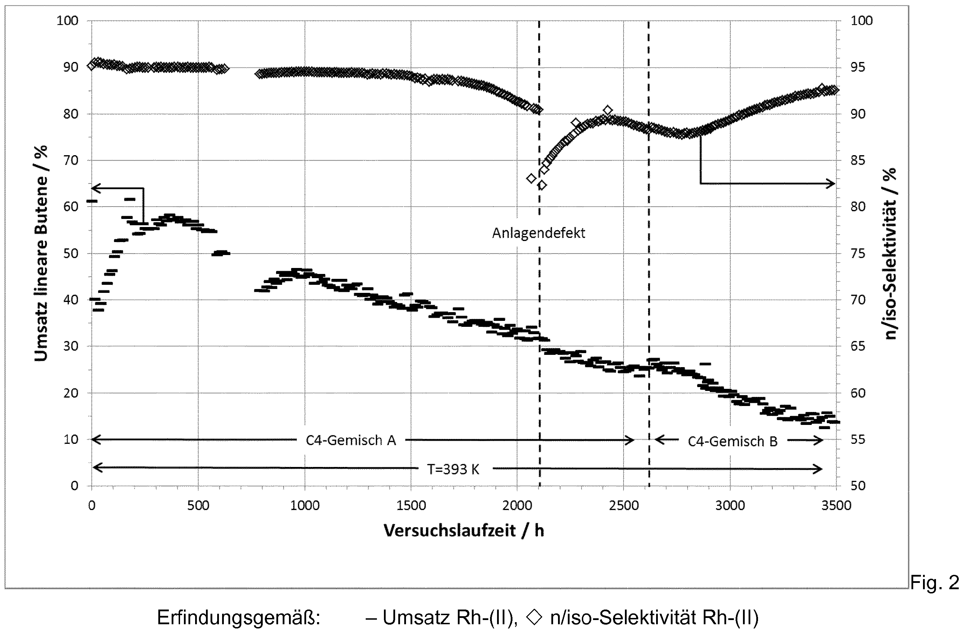

An Hand der Figuren Fig. 1 und Fig. 2 wird die vorliegenden Erfindung näher erläutert, ohne dass die Erfindung, deren Anwendungsbreite sich aus der gesamten Beschreibung und den Ansprüchen ergibt, auf die in den Beispielen genannten Ausführungsformen beschränkt sein soll.

Nicht erfindungsgemäß: Fig. 1 zeigt ein Umsatz-Zeit-Diagramm zur C4-Hydroformylierung mit Rh-(III) Katalysatoren (×) sowie die n/iso-Selektivität (○). Parameter: m_{Kat}=12g, m_{Rh}=0.2 Gew-%, L/Rh=10, ×,○: T=373,15-393,15K; p=1 MPa; p(1- und 2-Butene)=0,16 MPa (0,13 MPa); p(Butane)=0,06 MPa (0,05 MPa); p(H2)=p(CO)=0,39 MPa (0,41 MPa) vor (und nach) Variation des H2/1-Buten-Verhältnisses von 6 auf 8 nach 170 h Reaktionszeit, Verweilzeit=48s (43s). Zur besseren Übersichtlichkeit wurden von den ermittelten Messwerten gegenüber der Literatur (Schönweiz et al., Chem. Cat. Chem., 2013, DOI: 10.1002/cctc.201300305) nur jeder 10te gezeigt.

Erfindungsgemäß: In Fig. 2 wird ein Umsatz-Zeit-Diagramm zur C4-Hydroformylierung mit Rh-(II) Katalysatoren (-) sowie die n/iso-Selektivität (◇) wiedergegeben. Parameter: m_{Kat}=12 g, m_{Rh}=0.2 Gew-%, L/Rh=10, -,◇: T=393,15K; p=1 MPa; p(1- und 2-Butene)=0,16 MPa; p(Butane)=0,06 MPa; p(H2)=p(CO)=0,39 MPa, H2/1-Buten-Verhältnis = 6, Verweilzeit=58s. Zur besseren Übersichtlichkeit wurden von den ermittelten Messwerten nur jeder 10te gezeigt. 2106 h: kurzzeitiger Anlagenausfall aufgrund Defekts in der Anlage ohne Einbruch von Luftsauerstoff.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Alle nachfolgenden Präparationen wurden mit Standard-Schlenk-Technik unter Schutzgas durchgeführt. Die Lösungsmittel wurden vor Gebrauch, falls nicht anders vermerkt, über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Chemikalien

(Acetylacetonato)dicarbonylrhodium- in Kurzform (Rh(acac)(CO)₂) - und Dichlormethan (HPLC Reinheit) wurden ohne weitere Aufreinigung verwendet. Das mittelporige Siliziumdioxid ist kommerziell als Kieselgel 100 (0,2-0,5 mm) für die Säulen-Chromatographie bei der Firma Merck KGaA erhältlich. Siliziumdioxid wurde zur Herstellung der katalytisch aktiven Zusammensetzung 24 h bei 450°C kalziniert und anschließend weitere 24 h unter Vakuum bei 200 Pa gelagert. Weitere Lagerung des Siliziumdioxides erfolgte unter Argonathmosphäre. Der Ligand (II) wurde gemäß nachfolgenden Reaktionsschema hergestellt:

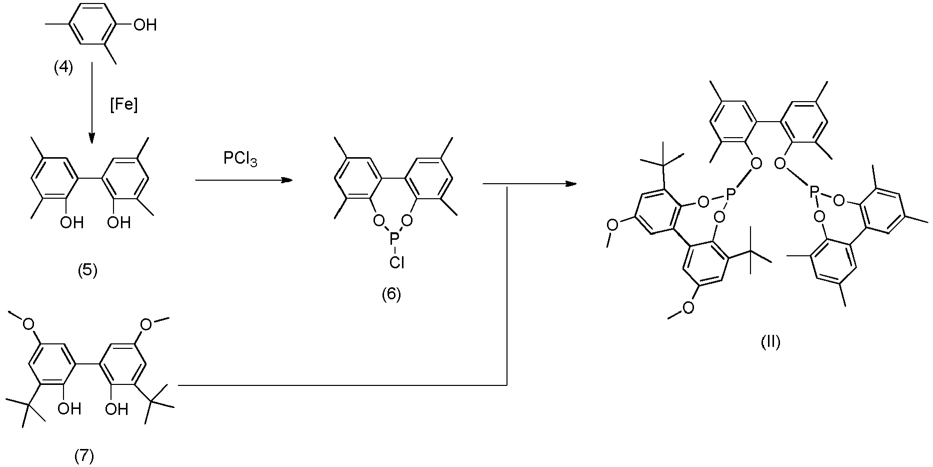

### Abkürzungen:

VE-Wasser = demineralisiertes Wasser
KPG = Kerngezogenes Präzisions-Glasgerät
ACN = Acetonitril
acac = acetylacetonat
NEt₃ = Triethylamin

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde der Gehalt des Liganden (II) bestimmt, wobei dieser unsymmetrische Ligand durch zwei Phosphorsignale charakterisiert wird.

### Synthese des 2,2'-Bis-(3,5-dimethylphenol)chlorophosphits (6)

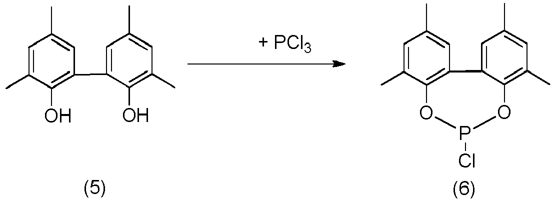

In einem sekurierten 2 L Schlenk mit Magnetrührer wurden 440 ml Phosphortrichlorid vorgelegt. In einem zweiten sekurierten 1 L Schlenk wurden 120 g 2,2' -Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Synthesevorschrift zur Herstellung des reinen Liganden (II)

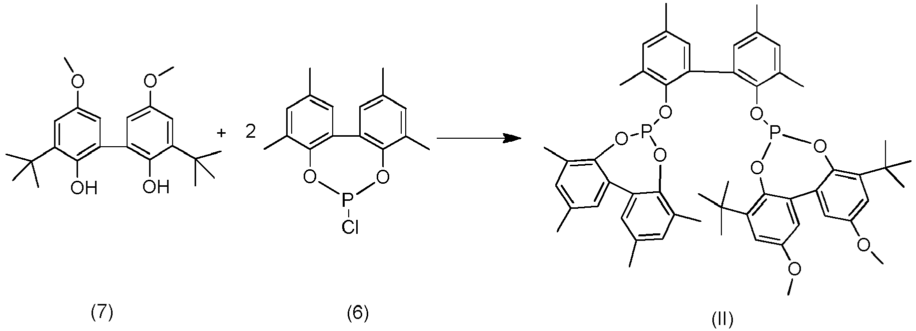

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem NEt₃ (0,29 mol) versetzt. Dann wurde langsam die Biphenol/ NEt₃-Lösung zu der Chlorophosphitlösung getropft. Nach einer Nachreaktionszeit von 1 h wurde die Reaktionslösung über Nacht bei 45°C gerührt.

Dieser Feststoff wurde in entgastem ACN 1.5h bei 75°C suspendiert und abgetrennt und mit warmen ACN nachgewaschen. Anschließend wurde das Produkt in Toluol 1.5h bei 35°C suspendiert und nachgewaschen. Das Zielprodukt (II) konnte als weißer Feststoff (33 g, 66%) erhalten werden. ³¹P-NMR (202,4 MHz, toluene-d₈): 142,5 und 140,9 (100%)

Die Herstellung des symmetrischen Bisphosphits Ligand (III) erfolgte gemäß DE 102006058682

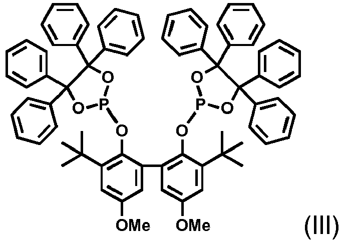

Synthesegas besteht aus einer Mischung mit einen Volumenanteil von 1 : 1 aus Wasserstoff und Kohlenmonoxid (≥ 99,97 %). Die technischen C4-Gemische besaßen die in Tabelle 2 angegebenen Zusammensetzungen:

**Tabelle 2:**

| | Zusammensetzung C4-Gemisch A / %^{[a]} Rh-(II): 0-2615 h | Zusammensetzung C4-Gemisch B / %^{[a]} Rh-(II): 2615-3506 h | Zusammensetzung C4-Gemisch C / %^{[a]} Rh-(III): 0-1195 h |
|---|---|---|---|
| 1-Buten + iso-Buten | 24,9 (24,8 + < 0,1) | 27 (26,9+< 0,1) | 27,8 (27,8+< 0,1) |
| Cis-2-Buten | 16 | 16 | 16 |
| Trans-2-Buten | 33 | 29 | 28 |
| n-Butan | 26 | 27,9 | 28 |
| Iso-Butan | < 0,1 | < 0,1 | < 0,1 |

| | | | |
|---|---|---|---|
| [a] GC-Fläche in % (Säule Fa. Agilent Technologies Länge 50 m, Innendurchmesser 0,32 mm, Filmdicke 0,5 µm, Trägergas Helium; Detektor: FID, Verdampfertemperatur 473,15 K, Split-Verhältnis 33,5:1, konstanter Säulenfluss Helium 91,6 ml min⁻¹, Detektortemperatur 523,15 K, Heizrampe: Anfangstemperatur 323,15 K, Haltezeit 15 min, Heizen auf 473,15 K mit 25 K min⁻¹, Haltezeit 40 min, Gesamtzeit pro Messung 61 min) | | | |

### Herstellung der katalytisch aktiven Zusammensetzung

Sämtliche Präparationen der katalytisch aktiven Zusammensetzung erfolgten mittels Schlenktechnik unter Argon (≥ 99.99 %). 0,40 mmol Rh(CO)₂(acac) wurde in ca. 160 ml Dichlormethan gelöst und für 10 Min. gerührt. Ein zehnfacher Überschuss des Liganden (II) (Molenverhältnis Ligand/Rhodium = 10) wurde zur Rhodium-Precursorlösung gegeben und 10 Min. gerührt. Anschließend wurde die benötigte Menge an kalziniertem Siliziumdioxid Kieselgel 100 (Massenverhältnis Rhodium/Trägermaterial = 0,2 %) zugesetzt. Die erhaltene Suspension wurde für 60 Min. gerührt. Dichlormethan wurde anschließend unter Vakuum an einem Rotationverdampfer bei 700 hPa und 40°C abgezogen, und das resultierende Pulver am Feinvakuum (40 Pa) über 24 h getrocknet. Bevor die katalytisch aktive Zusammensetzung zum Einsatz kam, wurde diese unter Argon-Atmosphäre gelagert. Die Präparation der katalytisch aktiven Zusammensetzung mit dem Liganden (III) erfolgt in Anlehnung an DE 102010041821, wobei auf die Zugabe der IL und als weitere Komponente, des organischen Amins, ausdrücklich verzichtet wird.

### Katalyse-Experimente

Sämtliche Hydroformylierungsversuche wurden in einem Festbettreaktor durchgeführt. Das trockene Katalysatormaterial wurde in den Rohrreaktor gefüllt und von beiden Seiten mit einem Stück Glaswolle fixiert. Die gesamt Anlage wurde bei Raumtemperatur mit Argon gespült und anschließend mit dem Reaktionsdruck (Argon) beaufschlagt. Falls kein Druckverlust festzustellen war, wurde der Reaktor in Argonstrom auf Reaktionstemperatur aufgeheizt. Nach Einstellen der jeweiligen Volumenströme wird für 4 Stunden Synthesegas (CO und H₂; Volumen = 1 zu 1, ≥ 99,97 %) durch den Reaktor geleitet. Die Zudosierung des Synthsesgases erfolgt durch Massendurchflussregler (Bezugsquelle Fa. Bronkhorst). Die Eduktdosierung der C4-Mischung erfolgte über eine HPLC-Pumpe (Bezugsquelle Fa. Knauer). In einem mit Glasperlen gefüllten Mischer wurde der Eduktgasstrom homogenisiert bevor dieser den Rohrreaktor samt Katalysatorschüttung von oben her durchströmte. Der Reaktor bestand aus rostfreiem Edelstahl (Durchmesser 12 mm, Länge 390 mm) und besaß an der Ausgangseite eine Gitter zur Positionierung des Katalysatormaterials. Durch ein innen liegendes Thermoelement konnte die Temperatur in der Katalysatorschüttung aufgezeichnet werden. Der Gesamtdruck in der Versuchsanlage wurde über ein elektronisches Druckhalteventil (Bezugsquelle Fa. Samson) geregelt. Auf der Niederdruckseite wurde der Produktgasstrom mit Hilfe eines Ventils aufgeteilt, sodass nur ein kleiner Anteil des Gesamtstromes zum Online-Gaschromatographen (Bezugsquelle Fa. Agilent, Modell 6890) geleitet wurde. Der größere Anteil wurde direkt in ein Produktfass geleitet. Durch ein Ventil wurden in regelmäßigen Zeitabständen Proben des Produktgasstroms in den Gaschromatographen injiziert. Die Datenauswertung erfolgte durch die ChemStation Software aus der Fa. Agilent.

### Analytik

Die Produktgaszusammensetzung während der Versuchslaufzeit wurde mit einem Online-Gaschromatographen analysiert. Der Gaschromatograph war ausgestattet mit einer Dimethylpolysiloxan beschichteten Säule (Fa. Agilent Technologies, Länge 50 m, Innendurchmesser 0,2 mm, Filmdicke 0,5 µm) und einem Flammenionisationsdetektor (FID). Eingestellte Messparameter: Injektortemperatur 423,15 K, Split-Verhältnis 33,5:1, konstanter Säulenfluss Helium 74 ml min⁻¹, Detektortemperatur 523,15 K, Heizrampe: Anfangstemperatur 323,15 K, Haltezeit 15 min, Heizen auf 473,15 K mit 25 K min⁻¹, Haltezeit 40 min, Gesamtzeit pro Messung 61 min.

### Ergebnisse

Der literaturbekannte, Benzpinakol-basierende Ligand (III), als katalytisch aktive Zusammensetzung Rh-(III), zeigt bei der Umsetzung mit technischen C4-Gemischen (Zusammensetzung siehe Tabelle 1, Spalte 3) eine sehr gute n/iso-Selektivität von > 90% mit einem Umsatz an linearen Butenen von anfänglich > 38 % (vergleiche Fig. 1). Nach 170 h Laufzeit ist bei einem hohen H₂/1-Buten-Verhältnis unter Reaktionsbedingungen von 8 zeigt die katalytisch aktive Zusammensetzung Rh-(III) eine typisch hohe n/iso-Selektivität von 99,4% zum linearen Aldehyd *n*-Pentanal. Diese hohen Selektivitätsergebnisse sind vergleichbar mit Resultaten für reines 1-Buten (Schönweiz et al., Chem. Cat. Chem., 2013, DOI: 10.1002/cctc.201300305) Nach Änderung des H₂/1-Buten-Verhältnises findet eine schleichende Desaktivierung über die betrachtete Reaktionszeit statt. Im Folgenden wurde eine Temperaturvariation bis zu einer Reaktionstemperatur von 120°C durchgeführt (vergleiche Fig. 1). Bei einer Temperatur von 120°C und einer Laufzeit von etwa 1000 h findet eine drastische Desaktivierung des Katalysatorsystems in ca. 200 h statt. Mit diesem Umsatzeinbruch fällt ebenso die n/iso-Selektivität auf 96% ab. Eine mögliche Erklärung der Desaktivierung kann die Bildung von Wasser als Nebenprodukt der Aldolkondensation aus den gebildeten Aldehyden sein. Ligand (III) als Bisphosphit neigt u.a. zur Alkoholyse.

Die erfindungsgemäße katalytisch aktive Zusammensetzung Rh-(II), mit dem unsymmetrisch substituierten Bisphosphit-Liganden (II), zeigt im Vergleich zu Rh-(III) eine etwas kleinere n/iso-Selektivität von durchschnittlich > 90% (vergleiche Fig. 2) aber einen Umsatz von > 55% nach 500 h. Dies liegt erstaunlicherweise über den erzielten Umsätzen von Rh-(III). Ebenso zeigt Rh-(II) eine deutlich längere Standzeit von mehr als 3500 h. Die Reaktionstemperatur lag über die gesamte Laufzeit von 3500 h bei 120°C. Der Reaktionsverlauf mit der katalytisch aktiven Zusammensetzung Rh-(II) zeigt nur einen schleichenden Rückgang des Umsatzes und keinen Einbruch im Umsatz, wie in Figur 1 mit dem System Rh-(III) dargestellt ist. Selbst nach einem Defekt in der Anlage ohne Einbruch von Luftsauerstoff bei 2106 h, regeneriert die n/iso-Selektivität der katalytisch aktiven Zusammensetzung Rh-(II) von < 83% wieder auf > 90%.

Dieses Ergebnis ist überraschend, da unsymmetrische substituierte Bisphosphite gegenüber symmetrisch substituierten deutlich an Selektivität einbüßen, wie bereits in Rhodium-catalyzed Hydroformylation, ed. by P.W.N.M. van Leeuwen et C. Claver, Kluwer Academic Publishers 2006, AA Dordrecht, NL, Seite 45-46 ausgeführt wird.. Des Weiteren zeigt sich die erfindungsgemäße Zusammensetzung Rh-(II) als deutlich temperaturstabiler als Rh-(III). Zudem deutet die Langzeitstabilität von Ligand (II) auf eine bessere Stabilität von (II) gegenüber Wasser hin. Durch die höhere Reaktionstemperatur der katalytisch aktiven Zusammensetzung Rh-(II) sollte die ebenfalls thermisch induzierte Aldolkondensation verstärkt gegenüber den Katalyseergebnissen von Rh-(III) im Versuchszeitraum 0 h bis 829 h sein. Der Ligand (II) in der katalytisch aktiven Zusammensetzung zeichnet sich also zum einen durch eine deutlich bessere Langzeitstabilität als alle bisher im Stand der Technik beschriebenen Liganden aus und zum anderen dadurch aus, dass er bei guten n/iso-Selektivitäten zusätzlich isomerisierend hydroformylieren kann und erfüllt somit die gestellte Aufgabe. Eine optimale Langzeitstabilität der katalytisch aktiven Zusammensetzung ist insbesondere in der großtechnischen Anwendung von Bedeutung, da durch die Heterogenisierung des Katalyseprozesses im Gegensatz zur in homogener Phase durchgeführten Hydroformylierung keine Nachdosierung dieser Zusammensetzung und ihrer Komponenten möglich sind.

## Patentansprüche

1. Zusammensetzung, umfassend:
a) mindestens ein Trägermaterial, welches porös ist;
b) mindestens ein Metall, ausgewählt aus der VIII, Nebengruppe des Periodensystems der Elemente;
c) mindestens eine Verbindung der Formel (II)

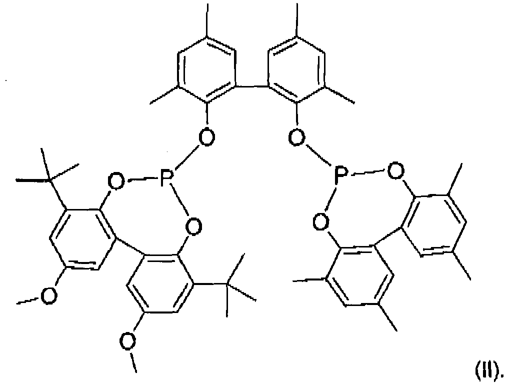

2. Zusammensetzung nach Anspruch 1, wobei das poröse Trägermaterial die Oberflächenparameter:
i) mittlerer Porendurchmesser in einem Bereich von 1 bis 423 nm;
ii) Porenvolumen in einem Bereich von 0,1 bis 2 ml/g;
iii) BET-Oberfläche in einem Bereich von 10 bis 2050 m²/g aufweist.

3. Zusammensetzung nach mindestens einem der Ansprüche 1 - 2, wobei das poröse Trägermaterial Oxide des Siliziums, Aluminiums, Titans, Zirkons oder Aktivkohle aufweist oder aus Mischungen davon besteht.

4. Zusammensetzung nach mindestens einem der Ansprüche 1 bis 3, wobei das Metall ausgewählt ist aus Cobalt, Rhodium, Iridium, Ruthenium.

5. Zusammensetzung nach Anspruch 4, wobei das Metall Rhodium ist.

6. Verfahren zur Herstellung einer Zusammensetzung nach den Ansprüchen 1 bis 5, welches die Schritte aufweist:
A) vorlegen einer Vorstufe mindestens einer Verbindung eines Metalls aus der VIII. Nebengruppe des Periodensystems der Elemente;
B) in-Kontakt-bringen mindestens einer Verbindung eines Metalls aus der VIII. Nebengruppe des Periodensystems der Elemente mit einem molaren Überschuß an mindestens einer Phosphorhaltigen organischen Verbindung der Formel (II) 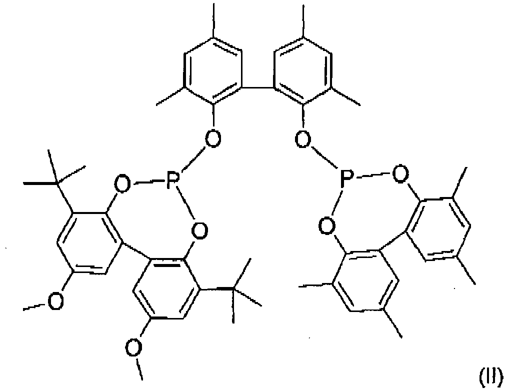 unter Verwendung eines inerten Lösungsmittels;
C) Zugabe mindestens eines porösen, inerten Trägermaterials zu der unter B) generierten Mischung;
D) entfernen des inerten Lösungsmittels unter Erhalt der Zusammensetzung.

7. Verwendung einer Zusammensetzung nach den Ansprüchen 1 bis 5 als Katalysator in einem Verfahren zur Hydroformylierung von ungesättigten Verbindungen.

8. Verfahren zu Hydroformylierung von ungesättigten Verbindungen, **dadurch gekennzeichnet, dass** eine Zusammensetzung nach den Ansprüchen 1 - 5, als Katalysator eingesetzt wird.

9. Verfahren nach Anspruch 8, **dadurch gekennzeichnet, dass** ein Festbettreaktor, enthaltend die Zusammensetzung verwendet wird.

10. Verfahren nach Anspruch 8 oder 9, **dadurch gekennzeichnet, dass** die ungesättigten Verbindungen ausgewählt sind aus C₂ - C₄ - Olefinen und deren technischen Gemischen.

11. Verfahren nach Anspruch 10, **dadurch gekennzeichnet, dass** die ungesättigten Verbindungen ausgewählt sind aus Ethen, Propen, Butenen oder deren technischen Gemischen.

12. Vorrichtung zur Hydroformylierung von ungesättigten Verbindungen welche als Festbettreaktor betrieben wird, **dadurch gekennzeichnet, dass** sie eine Zusammensetzung nach den Ansprüchen 1 - 5 aufweist.

## Claims

1. Composition comprising:
a) at least one support material which is porous;
b) at least one metal selected from transition group VIII of the Periodic Table of the Elements;
c) at least one compound of the formula (II)

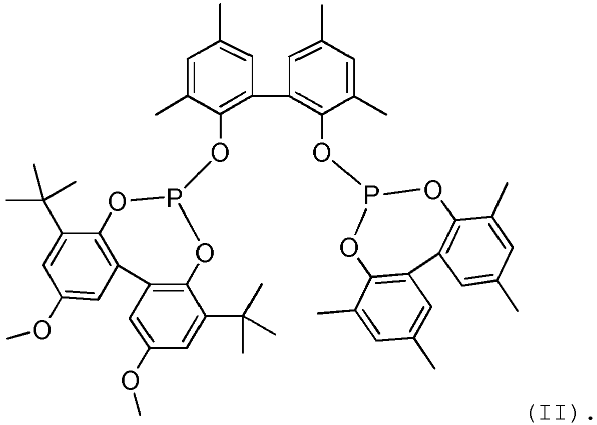

2. Composition according to Claim 1, wherein the porous support material has the following surface parameters:
i) mean pore diameter within a range from 1 to 423 nm;
ii) pore volume within a range from 0.1 to 2 mL/g;
iii) BET surface area within a range from 10 to 2050 m²/g.

3. Composition according to at least one of Claims 1-2, wherein the porous support material includes oxides of silicon, of aluminum, of titanium or of zirconium or activated carbon, or consists of mixtures thereof.

4. Composition according to at least one of Claims 1 to 3, wherein the metal is selected from cobalt, rhodium, iridium, ruthenium.

5. Composition according to Claim 4, wherein the metal is rhodium.

6. Process for producing a composition according to Claims 1 to 5, having the steps of:
A) initially charging a precursor of at least one compound of a metal from transition group VIII of the Periodic Table of the Elements;
B) contacting at least one compound of a metal from transition group VIII of the Periodic Table of the Elements with a molar excess of at least one organic phosphorus compound of the formula (II) 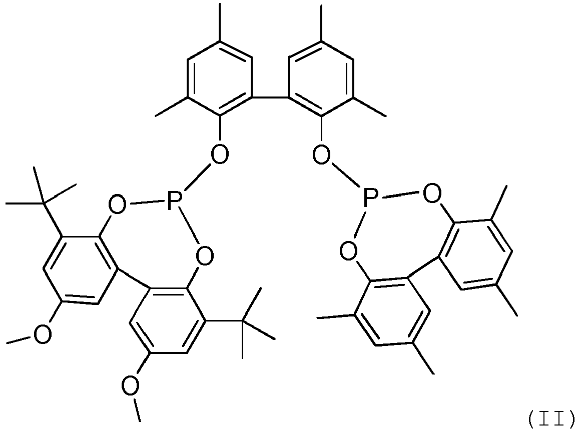 using an inert solvent;
C) adding at least one porous inert support material to the mixture generated in B);
D) removing the inert solvent to obtain the composition.

7. Use of a composition according to Claims 1 to 5 as catalyst in a process for hydroformylating unsaturated compounds.

8. Process for hydroformylating unsaturated compounds, **characterized in that** a composition according to Claims 1 to 5 is used as catalyst.

9. Process according to Claim 8, **characterized in that** a fixed bed reactor containing the composition is used.

10. Process according to Claim 8 or 9, **characterized in that** the unsaturated compounds are selected from C₂-C₄ olefins and the industrial mixtures thereof.

11. Process according to Claim 10, **characterized in that**
the unsaturated compounds are selected from ethene, propene, butenes and the industrial mixtures thereof.

12. Apparatus for hydroformylating unsaturated compounds
which is operated as a fixed bed reactor, **characterized in that** it has a composition according to Claims 1-5.

## Revendications

1. Composition, comprenant :
a) au moins un matériau support, qui est poreux ;
b) au moins un métal, choisi dans le groupe de transition VIII du tableau périodique des éléments ;
c) au moins un composé de formule (II)

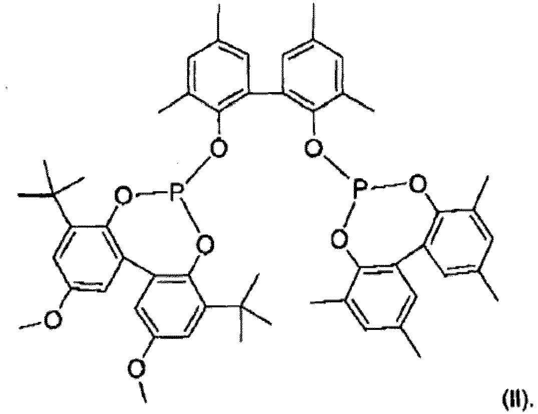

2. Composition selon la revendication 1, dans laquelle le matériau support poreux présente les paramètres de surface suivants :
i) un diamètre de pores moyen dans une plage allant de 1 à 423 nm ;
ii) un volume de pores dans une plage allant de 0,1 à 2 ml/g ;
iii) une surface BET dans une plage allant de 10 à 2 050 m²/g.

3. Composition selon au moins l'une quelconque des revendications 1 à 2, dans laquelle le matériau support poreux comprend des oxydes de silicium, d'aluminium, de titane, de zirconium ou du charbon actif ou est constitué de mélanges de ceux-ci.

4. Composition selon au moins l'une quelconque des revendications 1 à 3, dans laquelle le métal est choisi parmi le cobalt, le rhodium, l'iridium, le ruthénium.

5. Composition selon la revendication 4, dans laquelle le métal est le rhodium.

6. Procédé de fabrication d'une composition selon les revendications 1 à 5, qui comprend les étapes suivantes :
A) le chargement d'un précurseur d'au moins un composé d'un métal du groupe de transition VIII du tableau périodique des éléments ;
B) la mise en contact d'au moins un composé d'un métal du groupe de transition VIII du tableau périodique des éléments avec un excès molaire d'au moins un composé organique contenant du phosphore de formule (II) 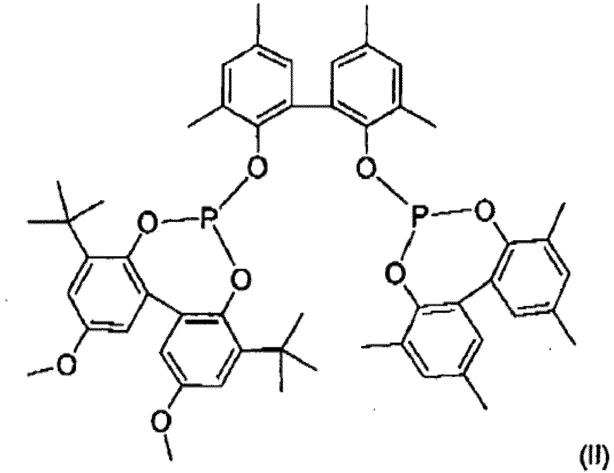 en utilisant un solvant inerte ;
C) l'ajout d'au moins un matériau support inerte poreux au mélange généré en B) ;
D) l'élimination du solvant inerte pour obtenir la composition.

7. Utilisation d'une composition selon les revendications 1 à 5 en tant que catalyseur dans un procédé d'hydroformylation de composés insaturés.

8. Procédé d'hydroformylation de composés insaturés, **caractérisé en ce qu'**une composition selon les revendications 1 à 5 est utilisée en tant que catalyseur.

9. Procédé selon la revendication 8, **caractérisé en ce qu'**un réacteur à lit fixe contenant la composition est utilisé.

10. Procédé selon la revendication 8 ou 9, **caractérisé en ce que** les composés insaturés sont choisis parmi les oléfines en C₂-C₄ et leurs mélanges techniques.

11. Procédé selon la revendication 10, **caractérisé en ce que** les composés insaturés sont choisis parmi l'éthène, le propène, les butènes ou leurs mélanges techniques.

12. Dispositif pour l'hydroformylation de composés insaturés, qui est exploité sous la forme d'un réacteur à lit fixe, **caractérisé en ce qu'**il comprend une composition selon les revendications 1 à 5.
